(19) Europäisches Patentamt
European Patent Office
Office européen des brevets

(11) **EP 3 349 655 B1**

(12) **EUROPEAN PATENT SPECIFICATION**

(45) Date of publication and mention of the grant of the patent:
**25.12.2019 Bulletin 2019/52**

(21) Application number: **16846715.7**

(22) Date of filing: **04.04.2016**

(51) Int Cl.:
*A61B 6/00* (2006.01)        *A61B 6/03* (2006.01)
*G06T 5/00* (2006.01)

(86) International application number:
**PCT/KR2016/003452**

(87) International publication number:
**WO 2017/047893 (23.03.2017 Gazette 2017/12)**

(54) **TOMOGRAPHY APPARATUS AND CONTROLLING METHOD FOR THE SAME**

TOMOGRAPHIEVORRICHTUNG UND STEUERUNGSVERFAHREN DAFÜR

APPAREIL DE TOMOGRAPHIE ET SON PROCÉDÉ DE COMMANDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.09.2015 KR 20150130334**
         **20.01.2016 KR 20160006771**

(43) Date of publication of application:
**25.07.2018 Bulletin 2018/30**

(73) Proprietor: **Samsung Electronics Co., Ltd.**
**Suwon-si, Gyeonggi-do 16677 (KR)**

(72) Inventors:
• **YI, Jong Hyon**
  **Yongin-si**
  **Gyeonggi-do 16903 (KR)**
• **RIFU, Toshihiro**
  **Suwon-si**
  **Gyeonggi-do 16543 (KR)**
• **AJAY, Narayanan**
  **Suwon-si**
  **Gyeonggi-do 16543 (KR)**
• **ZAMYATIN, Alexander**
  **Suwon-si**
  **Gyeonggi-do 16677 (KR)**

(74) Representative: **Gulde & Partner**
**Patent- und Rechtsanwaltskanzlei mbB**
**Wallstraße 58/59**
**10179 Berlin (DE)**

(56) References cited:
EP-A1- 3 326 533          US-A1- 2005 063 611
US-A1- 2006 247 513      US-A1- 2007 083 114
US-A1- 2007 230 653      US-A1- 2013 182 928
US-A1- 2014 328 528      US-B2- 7 929 746
US-B2- 8 923 644

**Description**

[Technical Field]

Embodiments of the present disclosure relate to a tomography apparatus and method for controlling the same.

[Background Art]

A medical imaging device is equipment for obtaining an image of an internal structure of an object. A medical image processing device is a non-invasive diagnostic device for scanning and processing structural details, internal tissues, and fluid flow in the body, and displaying them to the user. The user, e.g., a doctor may diagnose a health condition and illness of a patient using the medical image output from the medical image processing device.

As a typical device for scanning an object by X-ray radiation to a patient, there may be a Computed Tomography (CT) scanner.

The CT scanner corresponding to a tomography apparatus among various medical image processing devices is widely used for close examination of an illness, because it is capable of providing cross-sectional images of the object and represent the internal structure of e.g., an organ, such as kidneys, lungs, etc. in a non-overlapping way. A medical image obtained by the tomography apparatus is hereinafter referred to as a scanned image.

In obtaining the scanned image, the tomography apparatus is used to perform tomographic scanning on the object to obtain law data. Furthermore, the tomography apparatus performs certain pre-processing on the raw data to obtain projection data. The projection data may be a set of raw data scanned at one scanning angle. In other words, a set of raw data simultaneously obtained at the same scanning angle for all the channels is called the projection data.

As the tomography apparatus or an object subject to the tomographic scanning moves or due to the performance of the tomography device, blurring artifacts may be created in restoring the scanned image. For example, the blurring artifact may be created due to limits of performance, such as the size of a focal point of X-ray radiation, the size of an X-ray detector, the number of images obtained per rotation of the gantry, etc.

If the blurring artifact is created, the outermost edges of the object may be blurred and appear overlapping, and inner edges of the object in the scanned image appear blurred.

Such a blurring artifact in a scanned image degrades image quality of the scanned image. This may cause the user, e.g., a doctor, to read the image incorrectly and thus diagnose an illness inaccurately. Accordingly, when it comes to tomographic scanning, most important of all is to minimize the blurring artifact of a scanned image.

European patent application EP 3 326 533 A1 is directed to a tomography apparatus adapted to acquire a point spread function (PSF) according to a position in a field of view within the gantry. Furthermore, U.S. patent application US 2013/0182928 A1 discloses an image correction method for positron emission tomography (PET) images which includes estimating a PSF filter based on a detected edge of an object and de-blurring the PET image using the inverse PSF filter.

[Disclosure of Invention]

[Technical Problem]

An object of the present disclosure is to provide a tomography apparatus and method for controlling the same, which can reduce blurring artifacts that might be created in a restored scanned image. The invention is defined by the independent claims 1 and 10.

[Solution to Problem]

In accordance with an aspect of the present disclosure, a tomography apparatus is provided. The tomography apparatus includes a controller for estimating a Point Spread Function (PSF) corresponding to a location of an object; and an image processor for de-blurring projection data of the object based on the PSF corresponding to the location of the object.

The controller estimates a PSF corresponding to a distance between the object and an X-ray generator which is a radiation focal point.

The controller may estimate a PSF corresponding to a channel in which the object is located among a plurality of channels formed between an X-ray generator and an X-ray detector.

The controller may estimate the PSF based on projection data of a sample object and geometric information of the sample object.

The geometric information may include outline information of the sample object.

The image processor may perform de-blurring on projection data scanned at a rotation angle of a gantry.

The image processor may obtain a plurality of projection data corresponding to a plurality of rotation angles, and perform de-blurring on the projection data corresponding to each rotation angle.

The image processor may perform back projection based on the plurality of de-blurred projection data.

The controller may determine a distance between the X-ray generator and the object based on a cross-sectional image of a sample object scanned at a position.

The controller may determine a channel in which the object is located among a plurality of channels formed between the X-ray generator and the X-ray detector based on a cross-sectional image of a sample object scanned at a position.

The controller may estimate the PSF in the form of a Gaussian function.

The tomography apparatus may further include a storage for storing the PSF.

The storage may store a plurality of PSFs mapped to different positions of the object.

The image processor may generate a scanned image based on de-blurred projection data.

The tomography apparatus may further include a display for displaying the scanned image.

In accordance with another aspect of the present disclosure, a method for controlling a tomography apparatus is provided. The method includes estimating a Point Spread Function (PSF) corresponding to each location of an object; and de-blurring projection data of the object based on the PSF corresponding to the location of the object.

Estimating a PSF corresponding to each location of an object includes estimating a PSF corresponding to a distance between the object and an X-ray generator which is a radiation focal point.

Estimating a PSF corresponding to each location of an object may include estimating a PSF corresponding to a channel in which the object is located among a plurality of channels formed between an X-ray generator and an X-ray detector.

Estimating a PSF corresponding to each location of an object may include estimating the PSF based on projection data of a sample object and geometric information of the sample object.

De-blurring projection data of the object based on the PSF corresponding to the location of the object may include obtaining a plurality of projection data corresponding to a plurality of rotation angles, and performing de-blurring on the projection data corresponding to each rotation angle.

The method may further include performing back projection based on the plurality of de-blurred projection data.

Estimating a PSF corresponding to each location of an object may include determining a distance between the X-ray generator and the object based on a cross-sectional image of a sample object scanned at a position.

The tomography apparatus may further include an adaptive filter for correcting an PSF estimated by the controller, and the adaptive filter may correct the PSF depending on a location of a region of interest.

The PSF estimated by the controller may be a first PSF, and the adaptive filter may generate a second PSF based on a distance from a center of a Field of View (FOV) to the region of interest and a distance from a focal point of X-ray radiation.

The method may further include correcting the estimated PSF before de-blurring, and correcting the estimated PSF may include correcting the PSF depending on a location of a region of interest.

The estimated PSF may be a first PSF, and correcting the estimated PSF may include generating a second PSF based on a distance from a center of a Field of View (FOV) to the region of interest and a distance from a focal point of X-ray radiation.

[Advantageous Effects of Invention]

According to embodiments of the present disclosure of a tomography apparatus and method for controlling the same, blurring artifacts created within a scanned image may be effectively improved by creating a different PSF depending on a position of an object.

Furthermore, according to embodiments of the present disclosure of a tomography apparatus and method for controlling the same, the blurring artifact created within a scanned image may be more accurately eliminated by creating a PSF on projection data and performing de-blurring.

[Brief Description of Drawings]

FIG.1 is a schematic view of a Computed Tomography (CT) scanner;
FIG. 2 is a structure of a CT scanner, according to an embodiment of the present disclosure;
FIG. 3 shows an arrangement of a communication unit;
FIG. 4 shows views for explaining the need of accurate PSF estimation;

FIG. 5A is a block diagram of a tomography apparatus, according to an embodiment of the present disclosure;

FIGS. 5B and 5C are detailed views of an FOV area shown in FIG. 2;

FIG. 6 is a view for projection data of an object created at each rotation angle;

FIGS. 7 and 8 are views for explaining PSF;

FIG. 9 is a view for explaining how to estimate PSF;

FIG. 10 shows views for explaining a plurality of PSFs corresponding to a plurality of beam lines and bands;

FIG. 11 is a control block diagram of a tomography apparatus further including the adaptive filter, in accordance with another embodiment of the present disclosure;

FIG. 12 is a view for explaining a method for an adaptive filter to generate a second PSF;

FIG. 13 is a mimetic diagram of a sinogram, a set of a plurality of projection data;

FIG. 14 is a view for explaining a back projection process performed by an image processor;

FIG. 15 is a flowchart illustrating a method for controlling a tomography apparatus, according to an embodiment of the present disclosure;

FIG. 16 is a flowchart illustrating a method for controlling a tomography apparatus to explain operation of S1210 in detail; and

FIG. 17 is a flowchart illustrating a method for a tomography apparatus to perform image processing based on estimated PSFs.

[Best Mode for Carrying out the Invention]

Advantages, features, and methods for achieving them will be understood more clearly when the following embodiments are read with reference to the accompanying drawings. The embodiments of the disclosure may, however, be embodied in many different forms and should not be construed as limited to the embodiments set forth herein; rather, these embodiments are provided so that this disclosure will be thorough and complete, and will fully convey the scope of the embodiments of the disclosure to those skilled in the art. Like numbers refer to like elements throughout the specification.

Terms as used herein will be described before detailed description of embodiments of the present disclosure.

The terms are selected as common terms widely used now, taking into account principles of the present disclosure, which may however depend on intentions of ordinary people in the art, judicial precedents, emergence of new technologies, and the like. Some terms is selected at the inventor's discretion, in which case, description thereof will be explained later in detail. Therefore, the terms should be defined based on their meanings and descriptions throughout the specification of the present disclosure.

The term "include (or including)" or "comprise (or comprising)" is inclusive or openended and does not exclude additional, unrecited elements or method steps. Furthermore, the term 'unit' or 'module' refers to a software or hardware component, such as FPGA or ASIC which plays some role. However, the unit is not limited to software or hardware. The unit may be configured to be stored in an addressable storage medium, or to execute one or more processors. For example, the unit may include components, such as software components, object-oriented software components, class components and task components, processes, functions, attributes, procedures, subroutines, segments of program codes, drivers, firmware, microcodes, circuits, data, databases, data structures, tables, arrays, and variables. Functions served by components and units may be combined into a less number of components and units, or further divided into a more number of components and units.

Embodiments of the present disclosure will now be described in detail with reference to accompanying drawings to be readily practiced by an ordinary skill in the art. However, the present disclosure may be implemented in many different forms, and not limited to the embodiments as will be discussed herein. It should be noted that what is irrelative to the present disclosure is omitted from the drawings.

The term 'image' as herein used may refer to multi-dimensional data comprised of discrete image elements (e.g., pixels in a two dimensional (2D) image, and voxels in a three dimensional (3D) image). For example, an image may include a medical image of an object, which is obtained by a Computed Tomography (CT) scanner.

The term 'CT image' as herein used may refer to a composite image of a plurality of X-ray images obtained by scanning the object while rotating around at least one axis of the object.

The object may be a person or animal, or a part or all of the person or the animal. For example, the object may include at least one of organs, such as a liver, heart, uterus, breasts, abdomen, etc., and blood veins. The 'object' may also be a phantom. The phantom may refer to a substance having a density of a living thing and volume very close to an effective atomic number, and may include a spherical phantom having a similar nature to a body. The phantom may also include an image quality estimation phantom used for estimating a quality of an image, and a calibration phantom used for estimating a PSF.

The term 'user' as herein used may be a medical expert, e.g., a doctor, a nurse, a medical technologist, a medical image expert, etc., or a technician who fixes medical equipment, but is not limited thereto.

The tomography apparatus 100 may include any kind of tomography apparatuses, such as a CT scanner, an Optical Coherence Tomography (OCT), or Position Emission Tomography (PET) CT scanner.

A CT scanner will now be taken as an example of the tomography apparatus 100.

The CT scanner may provide relatively accurate cross-sectional images of an object by obtaining and processing image data, e.g., 2mm or less thick, hundreds times per second.

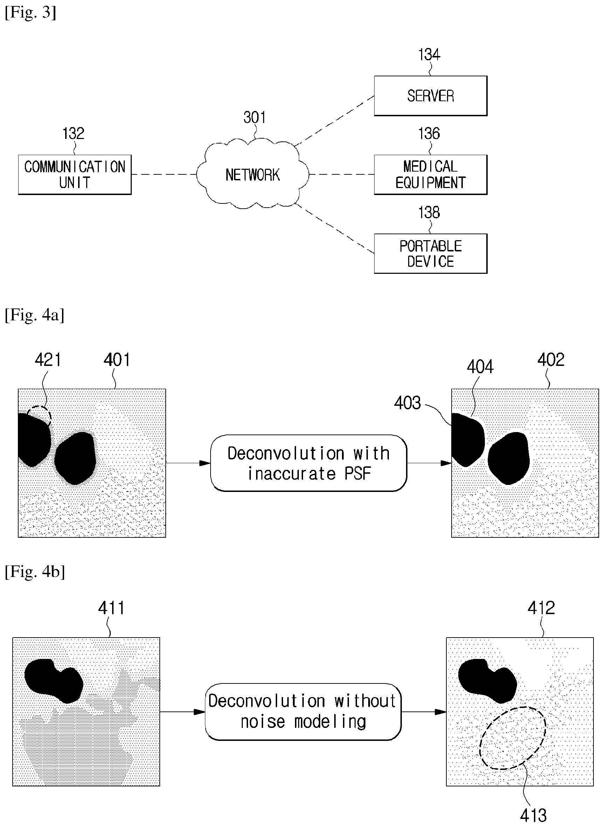

The CT scanner 100 in accordance with an embodiment of the present disclosure will be described with reference to FIG. 3. The CT scanner 100 may include various types of devices.

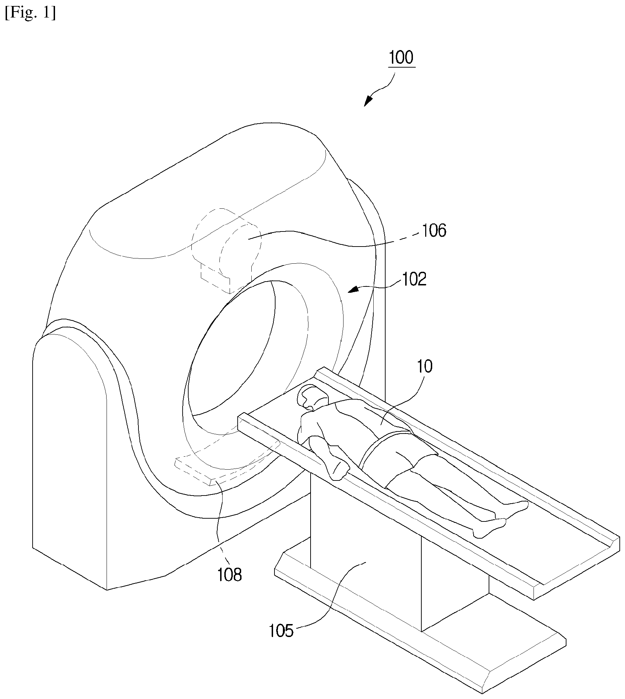

FIG.1 is a schematic view of a CT scanner.

Referring to FIG. 1, the CT scanner 100 may include a data obtainer 102, a table 105, an X-ray generator 106, and an X-ray detector 108.

The data obtainer 102 may be a gantry, and may include the X-ray generator 106 and the X-ray detector 108. The data obtainer 120 will now be described as the gantry.

The object 10 may be located on the table 105.

The table 105 may be moved in a certain direction (e.g., one of up, down, left, and right directions) in the process of CT scanning. Furthermore, the gantry 102 may also be inclined to a certain direction to an extent of a certain angle.

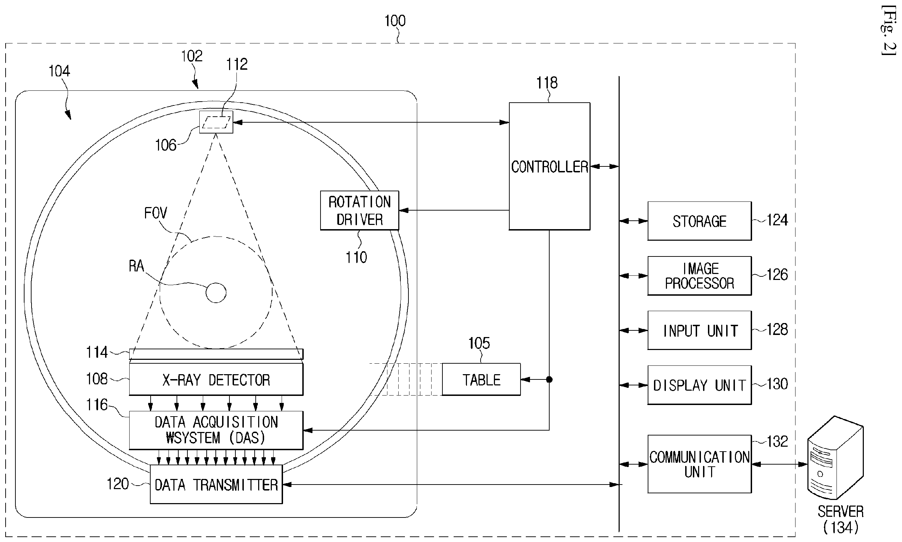

FIG. 2 is a structure of a CT scanner, according to an embodiment of the present disclosure.

The CT scanner 100 may include a gantry 102, a table 105, a controller 118, a storage 124, an image processor 126, an input unit 128, a display unit 130, and a communication unit 132.

As described above, the object 10 may be located on the table 105. The table 105 in accordance with an embodiment of the present disclosure is movable to a certain direction (e.g., one of up, down, left, and right directions), which may be controlled by the controller 118.

The gantry 102 in accordance with an embodiment of the present disclosure may include a rotating frame 104, the X-ray generator 106, the X-ray detector 108, a rotation driver 110, a data obtaining circuit 116, and a data transmitter 120.

The gantry 102 in accordance with an embodiment of the present disclosure may include the rotating frame 104 of a ring form that may be rotated around a rotation axis (RA). The rotating frame 104 may have the form of a disc as well.

The rotating frame 104 may include the X-ray generator 106 and X-ray detector 108 arranged to face each other to form a field of view (FOV). Furthermore, the rotating frame 104 may include an anti-scatter grid 114. The anti-scatter grid 114 may be located between the X-ray generator 106 and the X-ray detector 108. The FOV may be divided into a Scan Field of View (SFOV) that represents the entire scanning area from which the X-ray detector 108 may obtain an image and a Display Field of View (DFOV) that represents a partial area of the SFOV.

As for a medical imaging apparatus, X-ray radiation to reach a detector (or a sensitive film) includes not only attenuated primary radiation that forms a useful image but also scattered radiation that lowers image quality. To transmit the majority of the primary radiation and attenuate the scattered radiation, the anti-scatter grid may be located between the patient and the detector (or sensitive film).

For example, the anti-scatter grid may be constructed of alternating strips of lead foil and interspace materials such as solid polymer materials without cavity or solid polymers without cavity and fiber composite materials. However, the structure of the anti-scatter grid is not limited thereto.

The rotating frame 104 may receive a driving signal from the rotation driver 110, and rotate the X-ray generator 106 and X-ray detector 108 at a certain rotational speed. The rotating frame 104 may receive driving signals and power from the rotation driver 110 through a slip ring (not shown) in a way of contact. Alternatively, the rotating frame 104 may receive driving signals and power from the rotation driver 110 through wireless communication.

The X-ray generator 106 may receive a voltage or current from a Power Distribution Unit (PDU, not shown) through the slip ring and a high voltage generator (not shown) to irradiate X-rays. When the high voltage generator applies a certain voltage (hereinafter, referred to as a tube voltage), the X-ray generator 106 may generate X-rays having multiple energy bands in the spectrum to correspond to the certain tube voltage.

The X-rays generated by the X-ray generator 106 may be irradiated by a collimator 112 in a certain form.

The X-ray detector 108 may be located to face the X-ray generator 106. The X-ray detector 108 may include a plurality of X-ray detection elements. A single X-ray detection element may form a single channel, but is not limited thereto.

The X-ray detector 108 may detect the X-rays generated by the X-ray generator 106 and transmitted through the object 10, and generate an electric signal to correspond to the intensity of the detected X-rays.

The X-ray detector 108 may include an indirect-type detector for detecting radiation by converting the radiation to light, and a direct-type detector for detecting radiation by converting the radiation directly to charges. The indirect-type X-ray detector may use a scintillator. The direct-type X-ray detector may use a photon counting detector. A Data Acquisition System (DAS) 116 may be connected to the X-ray detector 108. An electric signal generated by the X-ray detector 108 may be collected by the DAS 116. The electric signal generated by the X-ray detector 108 may be wiredly or wirelessly collected by the DAS 116. Furthermore, the electric signal generated by the X-ray detector 108 may be provided to an analog-to-digital converter (not shown) through an amplifier (not shown). Data output from the X-ray detector 108 is called raw data.

Depending on the slice thickness or the number of slices, only a part of data collected from the X-ray detector 108 may be provided to an image processor 126, or the image processor 126 may select only a part of data.

Such a digital signal may be provided to the image processor 126 through the data transmitter 120. The digital signal may be wiredly or wirelessly transmitted to the image processor 126 through the data transmitter 120.

The controller 118 in accordance with an embodiment of the present disclosure may control operation of the respective modules of the CT scanner 100. For example, the controller 118 may control operation of the table 105, rotation driver 110, collimator 112, DAS 116, storage 124, image processor 126, input unit 128, display unit 130, communication unit 132, etc.

The image processor 126 may receive the data obtained from the DAS 116, e.g., data before processing, through the data transmitter 120, and perform pre-processing on the data.

Pre-processing may include, for example, a process of correcting non-uniform sensitivity between channels, or a process of correcting signal loss due to a drastic decrease in signal intensity or due to an X-ray absorbent like metal.

Resultant data pre-processed by the image processor 126 may be referred to as projection data. The projection data may be stored in the storage 124 together with scanning conditions in data acquisition (e.g., tube voltage, scanning angle, etc.).

The projection data may be a set of raw data scanned at one scanning angle. In other words, a set of raw data simultaneously obtained at the same scanning angle for all the channels is called the projection data.

The storage 124 may include storage media in at least one type of flash memory, hard disk, multimedia card micro type memory, card type memory (e.g., SD or XD memory), Random Access Memory (RAM), Static Random Access Memory (SRAM), Read-Only Memory (ROM), Electrically Erasable Programmable Read-Only Memory (EEPROM), Programmable Read-Only Memory (PROM), magnetic memory, magnetic disk, and optical disk.

The image processor 126 may reconstruct a cross-sectional image of an object using the obtained projection data. The scanned image may be a 3D image. In other words, the image processor 126 may create a 3D image of an object in e.g., a cone beam reconstruction method based on the obtained projection data.

External inputs, such as X-ray scanning conditions, image processing conditions, etc., may be received through the input unit 128. For example, X-ray scanning conditions may include a plurality of tube voltages, a plurality of X-ray energy value settings, a selection of scanning protocols, a selection of image reconstruction methods, FOV area settings, the number of slices, slice thickness, image post-processing parameter settings, etc. Image processing conditions may include the resolution of an image, attenuation coefficient settings of an image, image combination ratio settings, etc.

The input unit 128 may include e.g., a device for receiving certain inputs from outside. For example, the input unit 128 may include a microphone, a keyboard, a mouse, a joystick, a touch pad, a touch pen, a voice or gesture recognition device, etc.

The display unit 130 may display an X-ray scanned image reconstructed by the image processor 126.

Exchanges of data, power, etc., among the aforementioned elements may be performed using at least one of wired, wireless, and optical communications.

The communication unit 132 may perform communication with external devices, external medical devices, etc., through a server 134. This will be further described below in connection with FIG. 3.

FIG. 3 shows an arrangement of a communication unit.

The communication unit 132 may be connected to a network 301 via cable or wirelessly for communicating with the external server 134, a medical equipment 136, or a portable device 138. The communication unit 132 may exchange data with a hospital server or another medical equipment in the hospital through the Picture Archiving and Communication System (PACS).

Furthermore, the communication unit 132 may perform data communication with the portable device 138 according to the Digital Imaging and Communications in Medicine (DICOM) standard.

The communication unit 132 may transmit or receive data related to diagnosis of an object over the network 301. The communication unit 132 may also transmit or receive medical images obtained by the medical equipment 136, such as an MRI device, an X-ray scanner, etc.

Furthermore, the communication unit 132 may receive a patient's medical history or treatment schedule from the server 134 and use them for a clinical diagnosis of the patient. The communication unit 132 may further perform data communication not only with the server 134 or medical equipment 136 in the hospital but also with the portable device 138 of the user or the patient.

Moreover, the communication unit 132 may transmit information about whether an equipment is malfunctioned and about the state of quality management to a system manager or service person and receive feedback over a network.

All imaging devices have spatial resolution. The spatial resolution refers to precision of an image scanned by driving an imaging device to scan an object in a space. The image obtained from the imaging device may not perfectly represent a state of the object at the scanning point in time without blurring, due to the nature of the imaging device. For example, due to the movement of the imaging device itself while the imaging device is being driven, the movement may appear in a scanned image itself. Thus, the spatial resolution is determined by the extent of blurring that occurs in the image. For example, an imaging device having high spatial resolution has a less extent of blurring in its image compared to an imaging device having low spatial resolution.

The tomography apparatus has the spatial resolution as well. A limitation on the spatial resolution of the tomography apparatus causes blurring artifacts in a scanned image. Such a blurring artifact in a scanned image degrades image quality of the scanned image. This causes the user, e.g., a doctor, to read the image incorrectly and thus diagnose an illness inaccurately. For example, if a blurring artifact is created in a part of the image, which represents a calcium region, a blood vessel not actually clogged might look clogged. This may thus drop the accuracy in diagnosis of vascular disease.

The blurring artifact may be improved by estimating a Point Spread Function (PSF) and de-blurring the image based on the estimated PSF. The PSF varies depending on the tomography apparatus. Specifically, the PSF may vary depending on product specifications and/or performance of the tomography apparatus. The PSF has a complex form, and may vary with a position of the object formed within the gantry and a tube current measured in mill amperes for X-ray generation. Correcting the blurring artifact may be hereinafter referred to as de-blurring or de-blooming. For convenience of explanation, correcting the blurring artifact will now be collectively called 'de-blurring'.

In de-blurring an image based on a PSF, unless the PSF is accurately estimated, the image may be obtained with some blurred part not perfectly eliminated. Furthermore, in a de-blurred image, artifacts like undershooting or overshooting may be created.

In general, in estimating the PSF of the tomography apparatus, a single PSF is estimated and used. Specifically, a scanned image or reconstructed image is de-blurred by applying a single specified PSF for a particular tomography apparatus.

If an image is de-blurred based on the same PSF for all the positions within an FOV, the PSF may not be accurate for some positions. Furthermore, in case of de-blurring an image for which pre-processing and reconstruction has already been completed, inaccurate de-blurring may be performed on the entire image in that de-blurring is performed even on unnecessary information deleted or added in the step of reconstruction. Accordingly, an image may be obtained with some blurred part not perfectly eliminated, or some artifacts, such as undershooting or overshooting may be created in the image.

However, even within the FOV of the particular tomography apparatus, the PSF may vary depending on the position of the object. Furthermore, even with the projection data used in the step of image pre-processing, the PSF may be estimated and de-blurring may be performed.

Accordingly, in an embodiment of the present disclosure, the projection data is used to predict and use different PSFs depending on the position of an object formed within the gantry of the tomography apparatus. In other words, to estimate an accurate PSF, projection data is used to estimate a different PSF depending on the position of the object.

FIG. 4 shows views for explaining the need of accurate PSF estimation.

Referring to FIG. 4, initial images 401, 411 represent images before de-blurring. Parts that represent outlines of an object appear blurred in the initial image 401, due to blurring artifacts in the initial image 401. Referring to an area 421 shown in FIG. 4, outlines of the object are not represented with a clear gray level but with a plurality of gray levels having similar values, and thus the outline parts are not clearly imaged.

A de-blurred image 402 in FIG. 4 is an image de-blurred based on an inaccurate PSF. In this case, parts that represent outlines of an object 403 in the de-blurred image 402 appears clear compared to the initial image 401. However, an artifact in the form of a white band is created on parts 404 that represent outermost edges of the object 403.

Therefore, to effectively improve the blurring artifact in tomographic scanning of an object, a process of de-blurring an image based on an accurately estimated PSF is required.

In embodiments of the present disclosure, blurring artifacts created in an image may be effectively improved by de-blurring based on projection data and a different PSF depending on the position of the object formed within the gantry of a tomography apparatus.

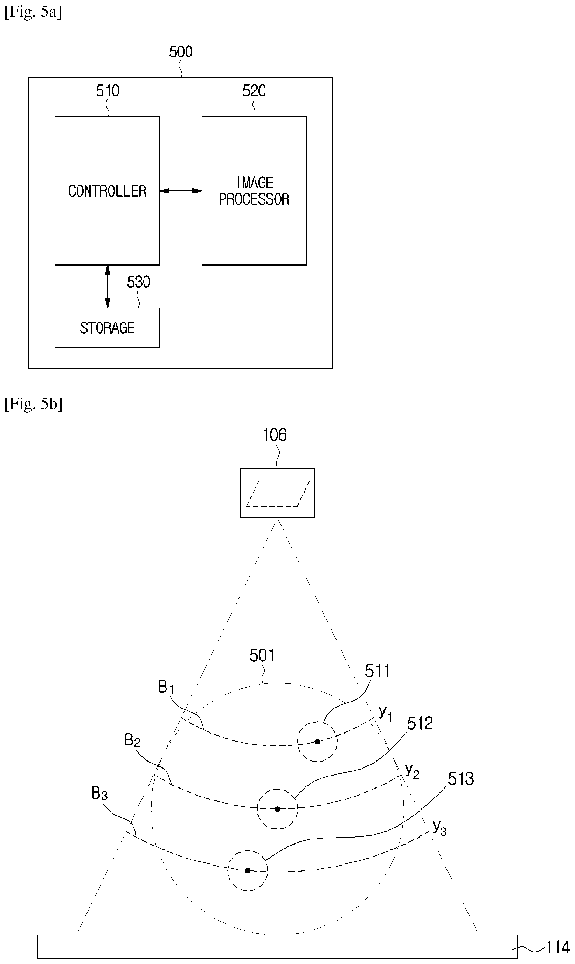

FIG. 5A is a block diagram of a tomography apparatus, according to an embodiment of the present disclosure.

Referring to FIG. 5A, a tomography apparatus 500 in accordance with an embodiment of the present disclosure includes a controller 510, an image processor 520, and a storage 530. The tomography apparatus 500 refers to any electronic device capable of performing tomographic scanning to obtain, reconstruct and/or display a scanned image.

The tomography apparatus 500 may be included in the CT scanner 100 as described above in connection with FIGS. 1 and 2. In this case, the controller 510, image processor 520, and storage 530 may correspond to the controller 118, image processor 126, and storage 124 shown in FIG. 2, respectively. Furthermore, the tomography apparatus 500 may be included in the medical equipment 136 or portable device 138 as described in FIG. 3, and may operate while being connected to the CT scanner 100.

The controller 510 in accordance with an embodiment of the present disclosure may obtain a PSF varying with the position within a FOV formed in the gantry.

The PSF may be fetched from outside of the tomography apparatus. Alternatively, in the tomography apparatus, a plurality of sample images may be obtained by scanning a sample object (e.g., a phantom) at a plurality of different positions, and a plurality of PSFs corresponding to the plurality of positions may be estimated.

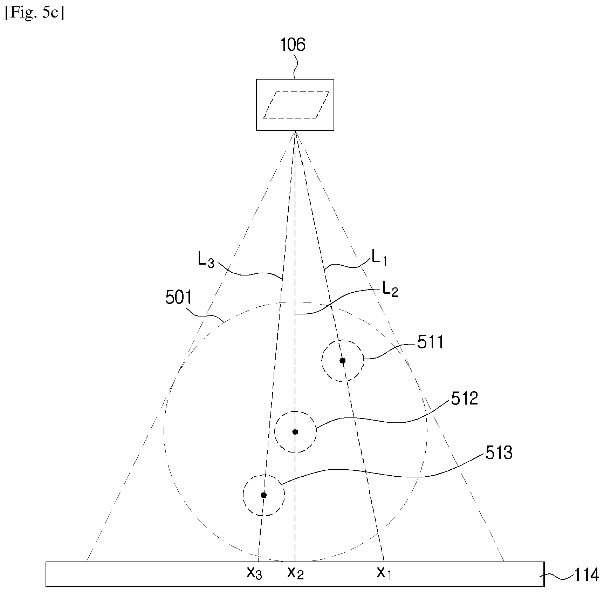

FIGS. 5B and 5C are detailed views of an FOV area shown in FIG. 2. In FIG. 5B, the same features as in FIG. 2 are represented with the same reference numerals.

Referring to FIGS. 5B and 5C, in an embodiment of the present disclosure, scanning is performed by placing a sample object at each of different positions 511, 512, 513 included within a FOV 501. Accordingly, the tomography apparatus 500 obtains a plurality of sample projection data for the plurality of different positions 511, 512, 513. Although a plurality of different positions are illustrated as the positions 511, 512, 513 in FIGS. 5B and 5C, they may be set to other various positions in addition to the illustrated positions in other embodiments. Furthermore, although the plurality of positions 511, 512, 513 at which the sample object may be located do not overlap each other in FIGS. 5B and 5C, some or all of the positions 511, 512, 513 may overlap in other embodiments.

Referring to FIG. 5B, the positions 511, 512, 513 of the sample object may be represented by distances from the X-ray generator 106 (i.e., distances from a radiation focal point). If points located at the same distance from the X-ray generator 106 in the FOV are defined to be in a single band, the position 511 of a first sample object, the position 512 of a second sample object, and the position 513 of a third sample object may be represented to correspond to a first band B1, a second band B2, and a third band B3, respectively.

The controller 510 may determine positions of the respective sample objects based on a plurality of sample scanned images corresponding to the plurality of bands, and estimate PSFs corresponding to the positions of the respective sample objects based on the sample projection data of the respective sample objects.

For example, when a sample object is located on the position 511 on the first band B1, the controller 510 determines that the sample object is located on the first band B1 using a first sample scanned image obtained by tomographic scanning, and estimates a first PSF for the first band B1 based on the first sample projection data.

Also, when a sample object is located on the position 512 on the second band B2, the controller 510 may determine that the sample object is located on the second band B2 using a second sample scanned image obtained by tomographic scanning, and estimate a second PSF for the second band B2 based on the second sample projection data. In the same way, the controller 510 may determine that a sample object is located on the third band B3 using a third sample scanned image and estimate a third PSF for the third band B3 based on the third sample projection data.

In this case, the positions 511, 512, 513 (on the bands B1, B2, B3) may be represented by y coordinates, and the controller 510 may estimate corresponding PSFs for the respective y coordinates.

Moreover, referring to FIG. 5C, the positions 511, 512, 513 of the sample objects may be represented by corresponding single device beam channel values. If points located at the same channel in the FOV are defined to be in a single beam line, the position 511 of the first sample object, the position 512 of the second sample object, and the position 513 of the third sample object may be represented to correspond to a first beam line L1, a second beam line L2, and a third beam line L3, respectively.

The controller 510 may determine positions of the respective sample objects based on a plurality of sample scanned images corresponding to the plurality of beam lines L1, L2, L3, and estimate PSFs corresponding to the positions of the respective sample objects based on the sample projection data of the respective sample objects.

For example, when a sample object is located at the position 511 in the first beam line L1, the controller 510 may determine that the sample object is located in the first beam line L1 using the first sample scanned image obtained by tomographic scanning, and estimate a fourth PSF for the first beam line L1 based on the first sample projection data, and when a sample object is located at the position 512 in the second beam line L2, the controller 510 may determine that the sample object is located in the second beam line L2 using the second sample scanned image obtained by tomographic scanning, and estimate a fifth PSF for the second beam line L2 based on the second sample projection data. In the same way, the controller 510 may determine that a sample object is located in the third beam line L3 using a third sample scanned image and estimate a sixth PSF for the third beam line L3 based on the third sample projection data.

In this case, the positions 511, 512, 513 (in the beam lines L1, L2, L3) may be represented by x coordinates, and the controller 510 may estimate corresponding PSFs for the respective x coordinates.

Moreover, referring both to FIGS. 5B and 5C, the positions 511, 512, 513 of the sample objects may be represented by distances from the X-ray generator 106 and single device beam channel values. The positions 511, 512, and 513 of the first, second, and third sample objects may be represented to correspond to the first band B1 and first beam line L1, the second band B2 and second beam line L2, and the third band B3 and third beam line L3, respectively.

The controller 510 may estimate a plurality of PSFs corresponding to a plurality of positions, based on a plurality of sample projection data corresponding to the plurality of bands B1, B2, B3 and plurality of beam lines L1, L2, L3.

In this case, the positions 511, 512, 513 of the first to third sample objects may be represented by combinations of x coordinates (x1, x2, x3) and y coordinates (y1, y2, y3), and the controller 510 may estimate corresponding PSFs for the respective xy coordinates.

Furthermore, the controller 510 may estimate a plurality of PSFs corresponding to beam angles formed by cone beams or fan beams.

Moreover, the controller 510 may estimate a PSF using interpolation or extrapolation even for a point at which no sample object is located.

In addition, in estimating PSFs, the controller 510 may estimate the PSFs in the form of a Gaussian function over distance. How to estimate the PSF, however, is not limited to the estimation in the form of the Gaussian function.

The controller 510 may estimate PSFs in a mathematical method as well. For example, the controller 510 may estimate PSFs by extracting edge information from projection data, differentiating the result of extraction, and performing Fourier transform.

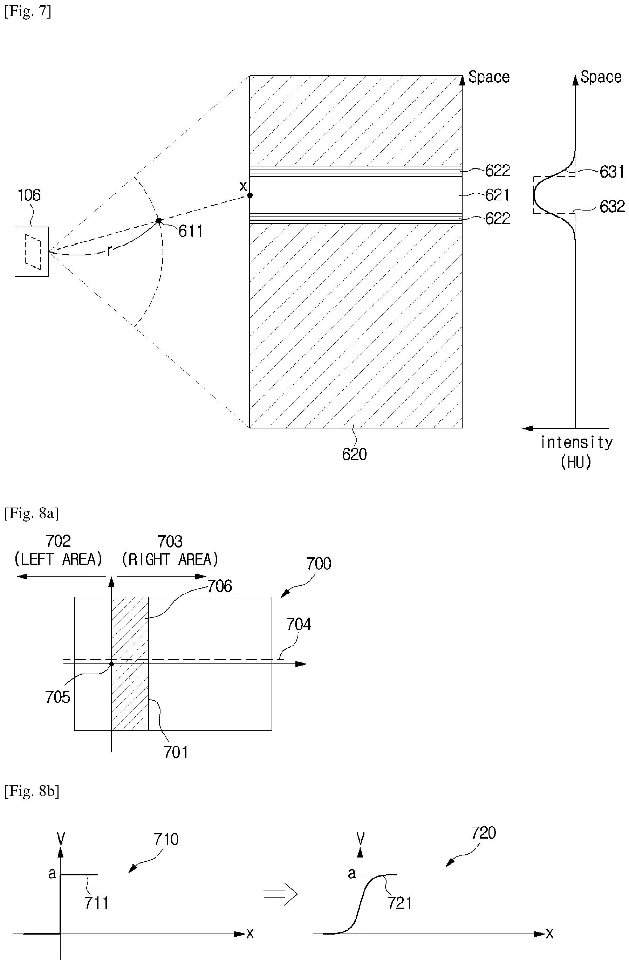

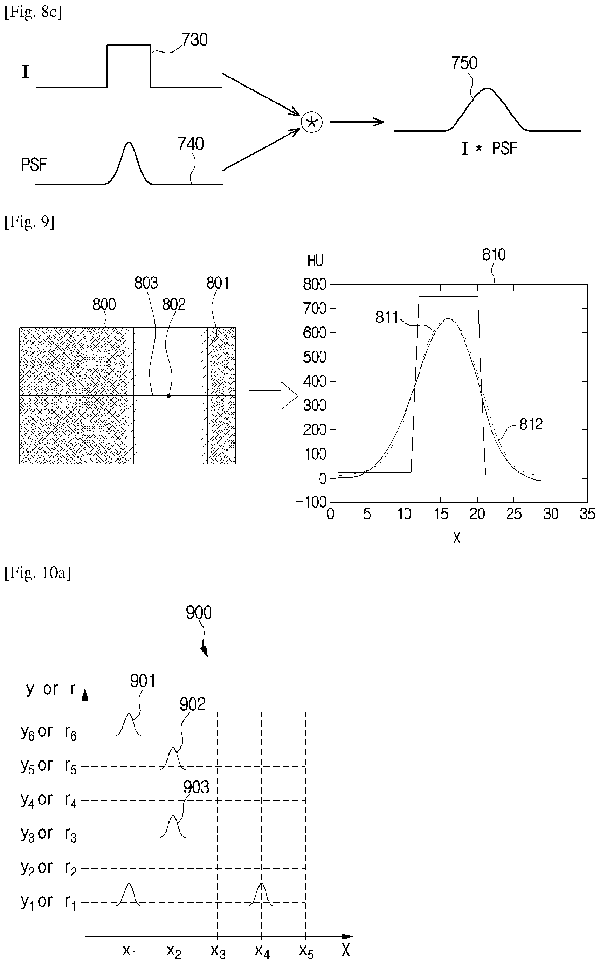

How to estimate the PSF will be described in detail later in connection with FIG. 8.

The plurality of PSFs estimated by the controller 510 may be stored in the storage 530. Specifically, the tomography apparatus 500 may store a plurality of PSFs in the storage 530 in advance. In this case, the storage 530 may store the PSFs corresponding to various geometric information. In this regard, the controller 510 may fetch a PSF for a position (in a band and beam line) of the object based on the plurality of PSFs stored in the storage 530.

A process of estimating and storing the plurality of PSFs in the storage 530 may be performed in the initial procedure of the tomography apparatus 500. Alternatively, the process may be performed in the process of calibration after internal parts of the tomography apparatus 500 are replaced. However, it is not limited thereto.

The storage 530 in accordance with an embodiment of the present disclosure may store the plurality of PSFs in the form of a table, but is not limited thereto.

The image processor 520 in accordance with an embodiment of the present disclosure may obtain projection data for which image pre-processing has been performed, by de-blurring projection data of an object scanned within the FOV based on PSFs corresponding to the respective positions, which are estimated by the controller 510. Specifically, the projection data may be de-blurred by performing deconvolution on the projection data based on estimated PSFs for the respective positions. De-blurring includes performing de-blurring by means of a Wiener filter. Deconvolution of an image is widely known to ordinary people in the art, so the description will be omitted herein.

The image processor 520 in accordance with an embodiment of the present disclosure may obtain projection data corresponding to the respective rotation angles by scanning the object at various angles along a rotating path of the gantry, and the aforementioned de-blurring may be performed on each projection data.

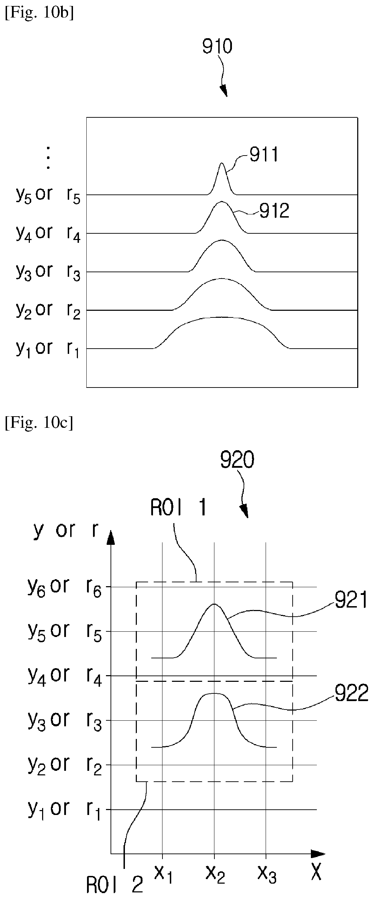

A process of performing de-blurring will be described later in connection with FIG. 10.

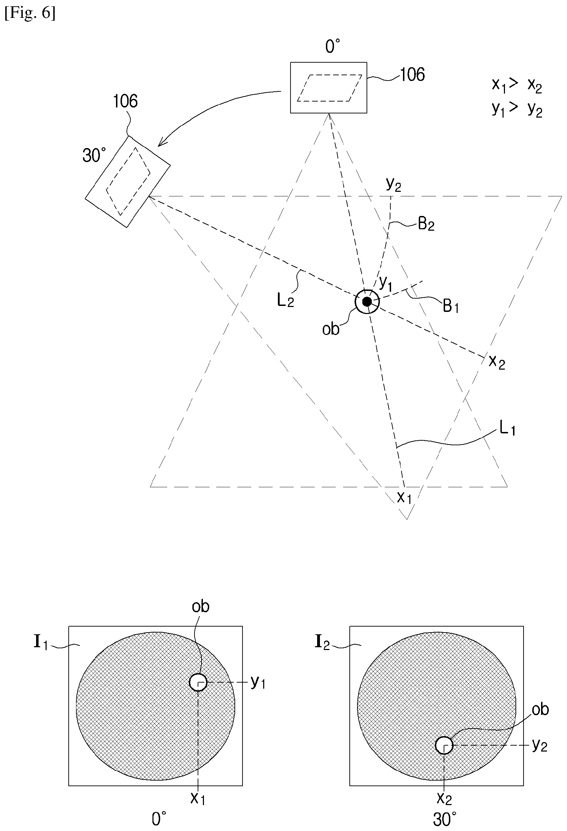

Referring to FIG. 6, a process for the controller 510 to determine a position of an object based on a cross-sectional image will be described in detail.

FIG. 6 is a view for cross-sectional images of an object, which are created at different rotation angles.

Referring to FIG. 6, when an object (ob) is located at a position within an FOV, the position 511 of the object (ob) may vary with the rotation angle of the gantry. In this case, the image processor 520 may obtain a first scanned image I1 corresponding to the rotation angle of 0 degree, and a second scanned image I2 corresponding to the rotation angle of 30 degrees. Of course, other scanned images corresponding to various rotation angles may be obtained. The first and second scanned images I1 and I2 may be cross-sectional images in which brightness level is represented in gray scale.

The controller 510 may determine a beam line and a band of the object (ob) at a rotation angle based on position information of the object (ob) appearing in the cross-sectional image.

Specifically, the controller 510 may determine x and y coordinates of a center position of an area in the first and second scanned images I1 and I2, which has brightness levels higher than a threshold to be a position of the object (ob).

The controller 510 may then determine a beam line and band information based on the x coordinate and the y coordinate, respectively, of the object (ob) that appears in the first scanned image I1. If a value x1 corresponds to the first beam line L1 and a value y1 corresponds to the first band B1, the controller 510 may determine that the object (ob) is located in the first beam line L1 and first band B1 at the rotation angle of 0 degree.

The controller 510 may also determine a beam line and a band based on the x coordinate and the y coordinate, respectively, of the object (ob) that appears in the second scanned image I2. If a value x2 corresponds to the second beam line L2 and a value y2 corresponds to the second band B2, the controller 510 may determine that the object (ob) is located in the second beam line L2 and second band B2 at the rotation angle of 30 degrees.

The image processor 520 may also obtain cross-sectional images at other rotation angles, and the controller 510 may determine beam lines and bands of the object (ob) corresponding to the respective rotation angles.

With the use of this beam line and band determination method, the image processor 520 may obtain cross-sectional images and projection data of a sample object located at different positions, and accordingly, the controller 510 may estimate PSFs corresponding to the respective positions of the sample object.

The PSF will now be described in more detail with reference to FIGS. 7 and 8.

FIG. 7 is a view for explaining PSF.

The PSF is a function that represents a spatial response of an image scanning device to a point. In other words, the PSF corresponds to a spatial impulse response of the image scanning device. The PSF herein may be approximated to a Gaussian function.

Referring to FIG. 7, a wave for imaging a point 611 of an object may be propagated from the X-ray generator 160. For example, a wave reaching the point 611 of the object may be irradiated from the point 611. As for a CT scanner, the wave may be an X-ray.

Such a wave may be obtained by an X-ray detector and represented on a projection data plane 620, in which case, since the point 611 of the object is not a point but has a definite area, it may be represented as an area 621 in the projection data plane 620 detected by the X-ray detector.

Furthermore, artifacts 622 may appear in the projection data plane 620. The artifacts 622 may be blurring artifacts.

FIG. 8 shows views for explaining PSF.

In FIG. 8, (a) shows an image 700 representing an area of an object that appears on the projection data plane. Arbitrary rectangular coordinates may be set in the image 700. For example, the x-axis may be set to go across an area 706 of an object while the $\gamma$-axis may be set to be adjacent to the area 706.

Referring to (b) of FIG. 8, the x-axis represents positions in a space, and v-axis represents pixel values for the respective positions. A graph 710 shown in (b) of FIG. 8 represents pixel values of a linear line 704 included in the image 700 when there is no blurring artifact created in the image 700.

In the graph 710 shown in (b) of FIG. 8, negative values of the x-coordinate correspond to a left area 702 while positive values of the x-coordinate correspond to a right area 703. The origin corresponds to a point 705 belonging to a surface 701. In this example of the graph 710, for negative x-coordinate values, pixel values are zero, while for positive x-coordinate values, pixel values correspond to 'a'. Accordingly, it can be seen that the image has sharp outlines when the x-coordinates are zero.

Furthermore, a graph 720 shown in (b) of FIG. 8 is obtained by transforming the graph 710 with a predefined PSF. The predefined PSF may represent a PSF that exists in the tomography apparatus.

In the graph 720, pixel values may gradually change around the x-coordinate of zero, due to the PSF of the tomography apparatus. Accordingly, the tomography apparatus may hardly obtain outlines from the linear image 720.

In FIG. 8, (c) shows views for explaining an effect of blurring artifacts.

Referring to (c) of FIG. 8, a graph 730 represents original brightness levels of the object without blurring artifact.

A graph 740 shown in (c) of FIG. 8 represents a PSF of the tomography apparatus. There is a blurring artifact in the graph 740, which appears in the form of an impulse.

As the tomography apparatus scans the object, the PSF of the tomography apparatus is applied, and projection data 750 with a blurring artifact may be obtained. Specifically, the tomography apparatus may obtain the graph 750 with the blurring artifact by convolution of the graph 730 and graph 740.

The tomography apparatus may obtain its PSF by obtaining the projection data 730 without blurring artifact and the projection data 750 with the PSF applied.

The projection data 730 without blurring artifact may be obtained by scanning, for example, with a thin substance, such as a thin wire or rod placed within the FOV.

There may be other various methods for estimating the PSF. For example, the tomography apparatus may have stored information about an original outline of the object (e.g., geometrical information), and may mathematically estimate the PSF based on the information. However, it is not limited thereto.

The tomography apparatus may receive information regarding the original form of the outline of the object from the outside of the apparatus. The information regarding the original form of the outline may be information having pixel values drastically changing around the x-coordinate of zero, as represented in the graph 710.

The tomography apparatus may also obtain the entire projection data from scanning of the object. The tomography apparatus may obtain a first area with less movement from the entire projection data. The tomography apparatus may obtain information regarding an outline of the object scanned in the first area. For example, the information regarding the outline of the scanned object may be information having pixel values slowly changing around the x-coordinate of zero, as represented in the graph 720. The tomography apparatus may estimate the PSF based on the information regarding the outline of the scanned object and the information regarding the original form of the outline.

Specifically, the PSF may be estimated by convolution of the inverse of the projection data 730, which is the information about the original form of the outline, and the projection data 750, which is the information about the outline of the scanned object.

How to estimate the PSF will now be described in detail with reference to FIG. 9. FIG. 9 is a view for explaining how to estimate a PSF.

Referring to FIG. 9, an image 800 is an image of projection data obtained by scanning a sample object having the form of a ball, rod, or bar. The horizontal axis and vertical axis of the image 800 represent positions of pixels that make up the image 800. For example, the image 800 may correspond to a 2D plane, which is an FOV plane. In this regard, due to the PSF of the tomography apparatus, blurring artifacts 801 may be created in the image 800.

The controller 510 in accordance with an embodiment of the present disclosure may measure a distribution of brightness levels with respect to a position 802 of a particular pixel that exists in a reference line 803 set in the image 800. Specifically, the brightness levels may be represented in Hounsfield Unit (HU) values. In this regard, the distribution 812 of HU values may appear in the form of a Gaussian function.

The controller 510 may estimate a PSF 811 at the particular position 802 based on the distribution 812 of HU values. The estimated PSF 811 may also appear in the form of a Gaussian function.

If the image processor 520 in accordance with an embodiment of the present disclosure obtains a plurality of sample projection data by scanning the sample object at a plurality of different positions (i.e., in a plurality of different beam lines and a plurality of different bands) within the gantry of the tomography apparatus, the controller 510 may determine the positions of the sample object and estimate PSFs for the respective positions of the sample object. A detailed method for determining the positions of the sample object was described above in connection with FIG. 6 and a detailed method for estimating the PSF was described above in connection with FIGS. 7 to 9, so the description of the methods will be omitted herein.

For example, the controller 510 may estimate a PSF corresponding to the first band and first beam line based on first sample projection data (P1, P1') of the sample data located in the first band and first beam line.

The controller 510 may also estimate a PSF corresponding to the second band and second beam line based on second sample projection data (P2, P2') of the sample data located in the second band and second beam line.

The projection data scanned in different beam lines and different bands may have the blurring artifacts 810 in different forms in the image. Accordingly, the distributions of HU values may appear differently, and the PSFs estimated based on the distribution of HU values may appear differently depending on the beam lines and the bands.

FIG. 10 shows views for explaining a plurality of PSFs corresponding to a plurality of beam lines and bands.

Referring to (a) of FIG. 10, a plurality of impulses 901, 902, 903 represented in a graph 900 indicate a plurality of PSFs estimated by the controller 510.

In (a) of FIG. 10, the horizontal axis corresponds to beam line information within an FOV, and the vertical axis corresponds to distances from the X-ray generator 106 within the FOV, i.e., distances r from a radiation focal point.

The more estimated PSFs there are, the more accurately the controller 510 may eliminate blurring artifacts based on the PSFs. The controller 510 may estimate a PSF using interpolation or extrapolation even for a point not actually measured.

Beam lines and bands for which PSFs are estimated may be changed by settings. Furthermore, beam lines and bands for which PSFs are estimated may be selected to cover as many areas within the gantry as possible.

Referring to (b) of FIG. 10, the controller 510 may generate a plurality of PSFs 911, 912 based only on information about the band, i.e., a distance r from the X-ray generator 106. In FIG. 10, (b) shows a graph 910 of the PSFs generated based on the band, with less amount of data to be stored as compared to (a) of FIG. 10.

In this case, as a plurality of different objects are scanned in the same band regardless of the beam lines, the same PSF may be applied to the plurality of objects during de-blurring.

Similarly, although not shown, the controller 510 may generate a plurality of PSFs 921, 922 based only on information x about beam lines, in which case, as a plurality of different objects are scanned in the same beam line regardless of the bands, the same PSF may be applied to the plurality of objects during de-blurring.

Furthermore, referring to (c) of FIG. 10, the controller 510 may generate the PSFs 921, 922 for each area by grouping the plurality of bands and plurality of beam lines.

In this case, the controller 510 may obtain representative PSFs 921, 922 of the grouped regions of interest ROI1, ROI2. Here, the representative PSF may be a PSF existing in the center of the region of interest. In performing de-blurring, for the objects located in fourth to sixth bands y4 to y6, and first to third beam lines x1 to x3, a PSF 921 for the fifth band y5 and the second beam line x2 is applied as a representative PSF of the first region of interest ROI1, and for the objects located in second to fourth bands y2 to y4, and first to third beam lines x1 to x3, a PSF 922 for the second band y2 and the second beam line x2 may be applied as a representative PSF of the second region of interest ROI2. De-blurring will be described in more detail later.

As such, once a PSF is estimated for each position, the estimated PSF may be stored in the storage 530 (see FIG. 5A) together with information about the position (band and beam line information).

In the meantime, once a PSF is estimated for each position, the tomography apparatus may further perform calibration to correct the estimated PSF according to the location of the region of interest (ROI).

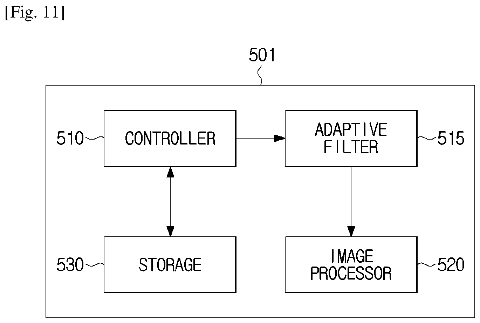

For this, the tomography apparatus in another embodiment of the present disclosure may further include an adaptive filter, and FIG. 11 is a control block diagram of a tomography apparatus further including the adaptive filter, in accordance with another embodiment of the present disclosure.

The tomography apparatus in accordance with another embodiment includes a controller 501, an adaptive filter 515, an image processor 520, and a storage 530.

The controller 501, image processor 520, and storage 530 are the same as the controller 501, image processor 520, and storage 530 described above in connection with FIG. 5A, so the overlapping description will be omitted.

If a PSF generated by the controller 510 is called a first PSF, the adaptive filter 515 corrects the first PSF according to the location of the region of interest ROI estimated by the controller 510.

For example, the adaptive filter 515 may generate a second PSF having the same or different value depending on a distance to the region of interest ROI from the isocenter (ISO) within or of an SFOV and a distance from the X-ray generator 106 (i.e., a distance from a radiation focal point), and generate a corrected PSF by convolution of the first PSF and the second PSF. The distance from the isocenter ISO of the region of interest ROI may correspond to a beam line located in the region of interest ROI, and the distance from the X-ray generator 106 may correspond to a band located in the region of interest ROI.

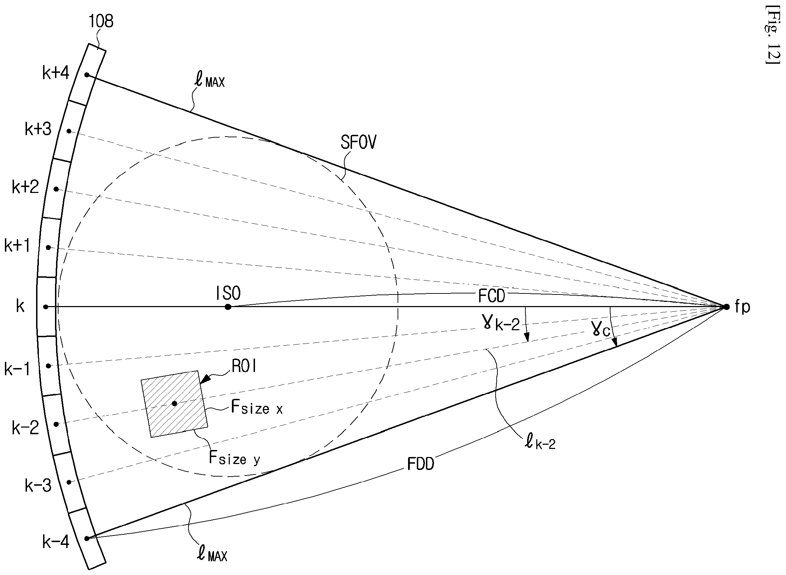

FIG. 12 is a view for explaining a method for an adaptive filter to generate a second PSF.

Let a point of the center of an SFOV be the isocenter ISO, a beam line in the SFOV that forms a maximum irradiation angle $\gamma_c$ from the optic axis $I_c$ of an X-ray irradiated by the X-ray generator 106 be a maximum angle beam line $I_{MAX}$, and an angle formed by a beam line $I_{k-2}$ corresponding to an X-ray element k-2 with respect to the optic axis be an element angle $\gamma_{k-2}$, the adaptive filter 515 may generate the second PSF in the following equation 1:

$$\mathrm{psf}(\mathrm{i}) = \exp\left(-\left(\frac{(\gamma i - \gamma c)^2}{2\sigma^2}\right)\right) \tag{1}$$

where i denotes a number of an X-ray detection element for receiving an X-ray from the X-ray generator 160, $\gamma_i$ denotes an element angle of a beam line corresponding to the $i^{th}$ detector element, $\gamma_c$ denotes a maximum irradiation angle, and $\sigma$ may be represented by the following equation 2:

$$\sigma^2 = \sigma_d^2 + \sigma_f^2$$

$$\sigma_d = \frac{DetSize}{2\sqrt{2\ln 2}} \frac{L}{FCD}$$

$$\sigma_f = \sigma_c \frac{FDD - L}{L}$$

$$\sigma_c = \sigma_a \cos^2 \gamma_C + \sigma_b \sin^2 \gamma_C$$

$$\sigma_a = \frac{a}{2\sqrt{2\ln 2}}, \qquad \sigma_b = \frac{b}{2\sqrt{2\ln 2}}$$

$$\tag{2}$$

where, a and b denote vertical and horizontal lengths of a region of interest ROI for which the second PSF is to be generated, respectively (see FIG. 12), DetSize denotes a detector pitch of one or more detector elements for detecting X-rays, FDD denotes a distance from an X-ray radiation focal point to a detector element of the X-ray detector 108, FCD denotes a distance from the X-ray radiation focal point (fp) of the X-ray generator 106 to the isocenter ISO, and L denotes a distance from the X-ray radiation focal point (fp) of the X-ray generator 106 to the region of interest ROI.

'a' and 'b' may be stored in the storage 530 in advance in the initial process or in the calibration process, or may have values set depending on the beam line and band, i.e., the region of interest ROI. For example, a may have a value of 1.2mm, and b may have a value of 8.1 mm.

As such, the adaptive filter 515 may generate the second PSF for each region of interest (ROI) and generate a corrected PSF by convolution of the first and second PSFs. The corrected PSF may be stored in the storage 530 and provided for the image processor 520 in de-blurring.

In the case of further including the adaptive filter 515, an image having uniform resolution for all the regions of interest within the SFOV may be obtained irregardless of the distance from the isocenter ISO.

The adaptive filter 515 may be implemented together with the image processor 520 in a single module. In this case, the image processor 520 may correct the PSF for each location of the region of interest ROI while performing de-blurring.

A method for the tomography apparatus to perform de-blurring will now be described.

The tomography apparatus may perform de-blurring on an image based on the stored PSF.

First, assuming that an object is located within an FOV, as described above in connection with FIG. 6, the controller 510 may determine a position where the object is located, i.e., a beam line and band, based on a cross-sectional image of the object scanned at a rotation angle.

The image processor 520 may then fetch a PSF corresponding to the beam line and band of the object from the storage 530, and mathematically calculate the inverse PSF based on the fetched PSF. The image processor 520 may estimate projection data without blurring artifact by convolution of the inverse PSF and projection data with a blurring artifact.

Alternatively, the image processor 520 may estimate projection data without blurring artifact by deconvolution of projection data with a blurring artifact and the PSF.

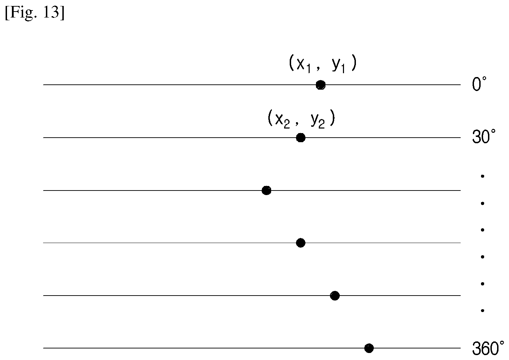

FIG. 13 is a mimetic diagram of a sinogram, a set of a plurality of projection data.

Given that a set of projection data is called a sinogram, referring to FIG. 13, the sinogram may include projection data in the range of rotation angles between 0 to 360 degrees.

The image processor 520 in accordance with an embodiment of the present disclosure may obtain projection data of an object at each rotation angle of the gantry, and perform de-blurring on the projection data based on the PSF corresponding to a position of the object, i.e., a beam line and band in which the object is located.

In this case, the image processor 520 may also perform de-blurring for a plurality of regions of interest (ROI) based on respective PSFs corresponding to the plurality of regions of interest ROI of the projection data scanned at a rotation angle of the gantry.

The PSF used in de-blurring may be the PSF before correction as described above in connection with FIG. 10, or the PSF after correction as described above in connection with FIG. 11.

De-blurring may be performed for all of the plurality of projection data corresponding to a plurality of rotation angles, e.g., 0, 10, ..., 350, 360 degrees. In other words, since corresponding beam lines x1, x2,... and bands y1, y2,... for the respective rotation angles are different, different PSFs may be applied for the respective rotation angles.

Furthermore, the image processor 520 may perform back projection based on the plurality of projection data, i.e., the sinogram, for which de-blurring has been performed. Back projection will further be described later. As a result of de-blurring and back projection, a scanned image with the blurring artifact eliminated may be produced.

The image processor 520 may also perform post image processing on the scanned image. For example, the image processor 520 may filter noise components from the scanned image.

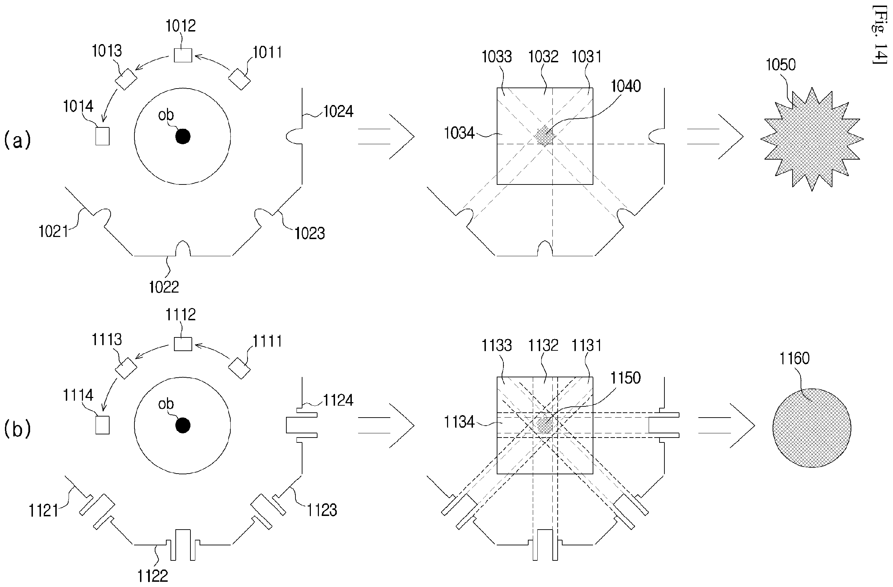

FIG. 14 is a view for explaining a back projection process performed by an image processor. An X-ray generator 1011 to 1014 shown in FIG. 14 is the same as the X-ray generator 160 as shown in FIG. 2.

The image processor 520 may perform back projection based on the plurality of projection data, i.e., the sinogram.

Referring to (a) of FIG. 14, as the X-ray generator 1011, 1012, 1013, 1014 irradiates X-rays onto an object (ob) at various rotation angles, projection data 1021, 1022, 1023, 1024 corresponding to the respective rotation angles may be generated. In case of representing the projection data 1021, 1022, 1023, 1024 as images 1031, 1032, 1033, 1034, an overlapping region 1040 is formed, and the region 1040 becomes a transmitted image 1050 corresponding to the object (ob). As shown in (a) of FIG. 14, if the image processor 520 performs back projection without performing de-blurring, the transmitted image 1050 of the object is not accurately represented due to blurring artifacts.

However, referring to (b) of FIG. 14, as the X-ray generator 1111, 1112, 1113, 1114 irradiates X-rays onto the object (ob) at various rotation angles, projection data corresponding to the respective rotation angles is created, and projection data (1121, 1122, 1123, 1124) for which de-blurring has been performed by applying different PSFs depending on the positions (i.e., bands and beam lines) of the respective projection data may be generated.

In case of representing the projection data 1121, 1122, 1123, 1124, for which de-blurring has been performed, as images 1131, 1132, 1133, 1134, an overlapping region 1140 is formed, and the region 1140 becomes a scanned image 1150 corresponding to the object (ob). As shown in (b) of FIG. 14, if the image processor performs back projection based on the projection data for which de-blurring has been performed, the scanned image 1150 of the object with the blurring artifact eliminated may be created.

In this regard, the image processor 520 may use a full reconstruction method to use scanned data obtained by one rotation of the X-ray generator 1111, 1112, 1113, 1114 to be reconstructed into the scanned image 1150. In addition, the image processor 520 may use a half reconstruction method to use scanned data obtained by more than half a rotation but less than one rotation of the X-ray generator 1111, 1112, 1113, 1114 to be reconstructed into the scanned image 1150. The method for reconstructing a scanned image, however, is not limited thereto.

Subsequently, the image processor 520 in accordance with an embodiment of the present disclosure may filter noise components from the scanned image 1150. Filtering the noise components may employ noise filtering methods commonly known to the ordinary people in the art, so the description will be omitted herein.

A display unit may output a screen containing a final scanned image obtained by the image processor 520. The display unit may also display a user interface screen required to proceed scanning.

The display unit corresponds to the display unit 130 shown in FIG. 2, so the overlapping description will be omitted.

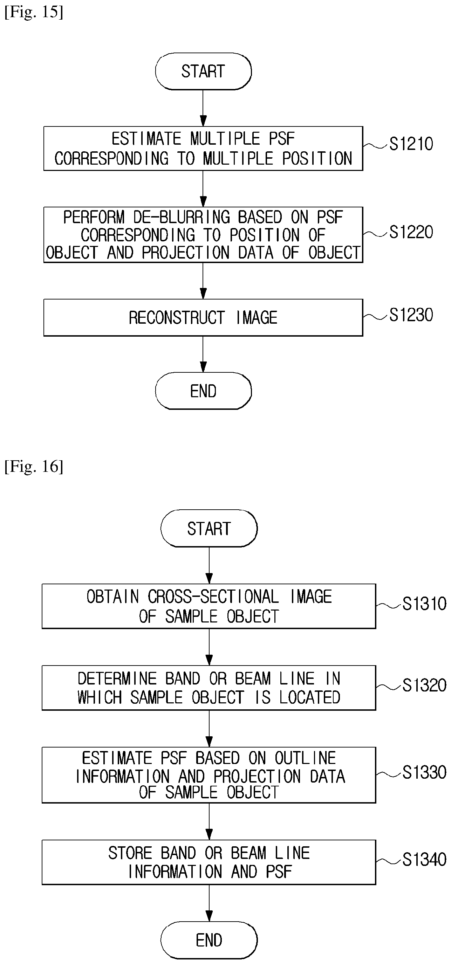

FIG. 15 is a flowchart illustrating a method for controlling a tomography apparatus, according to an embodiment of the present disclosure.

Operational features of the method for controlling the tomography apparatus in accordance with an embodiment of the present disclosure are the same as those of the tomography apparatus 100, 500 as described above with reference to FIGS. 1 to 14. Accordingly, in explaining the method for controlling the tomography apparatus, the overlapping descriptions with those of FIGS. 1 to 14 will be omitted.

Referring to FIG. 15, in the method for controlling a tomography apparatus in accordance with an embodiment of the present disclosure, a plurality of PSFs corresponding to a plurality of positions are estimated based on a plurality of sample projection data obtained by scanning a sample object at the plurality of different positions, in step S1210. The operation of step S1210 may be performed by the controller of the tomography apparatus according to an embodiment of the present disclosure. In this case, the position of the sample object may be manually input by the user, or the controller may directly determine the position based on a cross-section image (see FIG. 6).

In the method for controlling a tomography apparatus in accordance with an embodiment of the present disclosure, de-blurring is performed based on the PSFs corresponding to the respective positions of the object and the projection data obtained by scanning the object, in step S1220. In this case, the position of the object may also be manually input by the user, or the controller may directly determine the position based on a cross-sectional image (see FIG. 6).

Specifically, performing deconvolution on the projection data of the object based on the generated PSF, a blurring artifact present in the projection data of the object may be improved. The operation of step S1220 may be performed by the image processor of the tomography apparatus according to an embodiment of the present disclosure.

In the method for controlling a tomography apparatus in accordance with an embodiment of the present disclosure, a final scanned image is obtained by performing image reconstruction, such as performing back projection based on a plurality of de-blurred projection data, i.e., a sinogram, and filtering the noise components, in step S1230. The plurality of de-blurred projection data may be ones scanned at different rotation angles of the gantry 102 of the tomography apparatus 100. The operation of step S1230 may be performed by the image processor of the tomography apparatus according to an embodiment of the present disclosure.

FIG. 16 is a flowchart illustrating a method for controlling a tomography apparatus to explain operation of S1210 in detail.

Referring to FIG. 16, in the method for controlling a tomography apparatus in accordance with an embodiment of the present disclosure, a plurality of sample projection data are obtained by scanning a sample object at a plurality of different positions, in step S1310. In this case, the plurality of different positions may each be defined with one of a plurality of bands and one of a plurality of beam lines.

A band refers to a line formed by connecting positions at the same distance from the X-ray generator 106, and a beam line refers to a line formed by connecting positions located on the same channel (i.e., on the same beam path) within the FOV.

The operation of step S1310 may be performed by the controller of the tomography apparatus according to an embodiment of the present disclosure.

In the method for controlling a tomography apparatus in accordance with an embodiment of the present disclosure, a band or beam line in which the sample object is located is determined based on a cross-sectional image of the sample object, in step S1310.

The beam line or band in which the sample object is located may be determined based on a relative location of a bright region compared to the entire region in the cross-sectional image of the sample object.

The operation of step S1320 may be performed by the controller of the tomography apparatus according to an embodiment of the present disclosure.

In the method for controlling a tomography apparatus in accordance with an embodiment of the present disclosure, a PSF of the sample object is estimated based on outline information of the sample object and projection data of the sample object, in step S1330.

The outline information of the sample object is information regarding an original form of the sample object, e.g., information fetched from outside of the tomography apparatus.

Specifically, in the method for controlling a tomography apparatus, a PSF may be estimated by convolution of the inverse of projection data regarding the original form of the outline and projection data regarding the scanned outline of the object.

The operation of step S1330 may be performed by the controller of the tomography apparatus according to an embodiment of the present disclosure.

In the method for controlling a tomography apparatus in accordance with an embodiment of the present disclosure, the information about the band or beam line of the estimated PSF is stored with the estimated PSF, in step S1340.

For example, information about a band corresponding to the estimated PSF among the plurality of bands and the estimated PSF may be mapped and stored. Furthermore, information about a beam line corresponding to the estimated PSF among the plurality of beam lines and the estimated PSF may be mapped and stored. Moreover, the information about a band corresponding to the estimated PSF among the plurality of bands and the information about a beam line corresponding to the the estimated PSF among the plurality of beam lines are mapped together to the estimated PSF and stored.

The operation of step S1340 may be performed by the controller of the tomography apparatus according to an embodiment of the present disclosure, and the information about the band or beam line for the estimated PSF may be stored with the estimated PSF in the storage.

In another embodiment, the tomography apparatus may further include an adaptive filter, and the method for controlling a tomography apparatus in accordance with an embodiment of the present disclosure may further include correcting PSFs for different locations of regions of interest ROI estimated by the controller, i.e., different locations of the sample object. This was described above in connection with FIGS. 11 and 12, so the description is omitted herein.

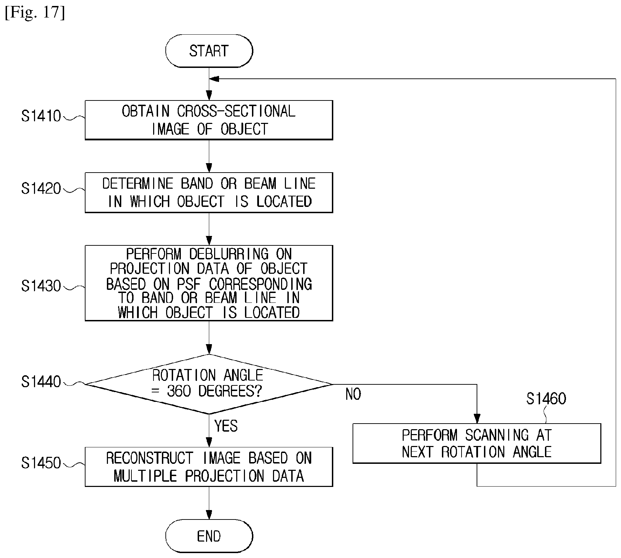

FIG. 17 is a flowchart illustrating a method for a tomography apparatus to perform image processing based on estimated PSFs.

Referring to FIG. 17, in a method for controlling a tomography apparatus in accordance with an embodiment of the present disclosure, a cross-sectional image of an object scanned at a position is obtained, in step S1410. The image processor of the tomography apparatus may obtain a cross-sectional image at the rotation angle of 0 degree.

The operation of step S1410 may be performed by the image processor of the tomography apparatus according to an embodiment of the present disclosure.

In the method for controlling a tomography apparatus in accordance with an embodiment of the present disclosure, a band or beam line in which the object is located is determined based on the obtained cross-sectional image of the object, in step S1420.

In the method, the beam line or band in which the object is located may be determined based on a relative location of a bright region compared to the entire region in the cross-sectional image.

The operation of step S1420 may be performed by the controller of the tomography apparatus according to an embodiment of the present disclosure.

In the method for controlling a tomography apparatus in accordance with an embodiment of the present disclosure, projection data of the object is de-blurred based on the determined band or beam line in which the object is located, in step S1430. The projection data may be obtained by the image processor.

Specifically, in the method for controlling a tomography apparatus in accordance with an embodiment of the present disclosure, a blurring artifact created in the projection data may be improved by fetching a PSF from the storage corresponding to the determined band or beam line in which the object is located and performing deconvolution on the projection data of the object based on the PSF.

The operation of step S1430 may be performed by the image processor of the tomography apparatus according to an embodiment of the present disclosure.

Although in the aforementioned embodiments, de-blurring is performed only for the region of interest in which the object is located, de-blurring may be performed even for the other regions of interest where the object is not located.

Furthermore, although in the aforementioned embodiments, the adaptive filter corrects the PSF depending on the different location of the region of interest ROI estimated by the controller, and the image processor performs de-blurring using the corrected PSF, it is also possible for the image processor to correct the PSF generated by the controller while performing de-blurring. In this case, the image processor may correct the PSF depending on the location of the region of interest.

In the method for controlling a tomography apparatus in accordance with an embodiment of the present disclosure, projection data of the object is obtained again at different rotation angles in step S1460 until the X-ray generator completes a full round turn, and de-blurring is performed on the projection data using the PSF based on a relative position (i.e., a band or beam line) of the object, which varies with the respective rotation angles.

Although step S1440 was described as using the full reconstruction method by the tomography apparatus, the half reconstruction method may be used as well, in which case projection data of the object may be obtained until the X-ray generator turns half a round, i.e., 180-degree turn.

In the method for controlling a tomography apparatus in accordance with an embodiment of the present disclosure, in case of obtaining a plurality of de-blurred project data, i.e., sinogram at a plurality of rotation angles, an image is reconstructed based on the plurality of de-blurred projection data, in step S1450.

For example, in the method for controlling a tomography apparatus, noise components may be filtered from the scanned image.

The operation of step S1450 may be performed by the image processor of the tomography apparatus according to an embodiment of the present disclosure.

According to embodiments of the present disclosure of a tomography apparatus and method for controlling the same, blurring artifacts created within a scanned image may be effectively improved by generating different PSFs depending on the position of an object.

Furthermore, according to embodiments of the present disclosure of a tomography apparatus and method for controlling the same, the blurring artifact created within the scanned image may be accurately eliminated by generating a PSF and performing de-blurring on projection data.

The aforementioned embodiments of the present disclosure may be written into a program that may be executed by a computer, and may be implemented in a universal digital computer for carrying out the program using a computer-readable recording medium.

The computer-readable recording medium includes a storage medium, such as magnetic storage medium (e.g., ROM, flopy disk, hard disk, etc.), an optical meium (e.g., CD-ROM, DVD, etc.), and carrier waves (e.g., transmission over the Internet).

Embodiments of the present disclosure have been described with reference to accompanying drawings, but it is to be understood that various modifications can be made without departing the scope of the present invention.

While the disclosure has been shown and described with reference to certain exemplary embodiments thereof, it will be understood by those skilled in the art that various changes in form and details may be made therein without departing from the scope of the disclosure as defined by the appended claims.

## Claims

1. A tomography apparatus (100) comprising:

   a controller (118) for estimating a Point Spread Function (PSF) corresponding to a location of an object (10); and an image processor (126) for de-blurring projection data of the object (10) based on the PSF corresponding to the location of the object (10), **characterised in that** the controller (118) is configured to estimate the PSF corresponding to a distance between the object and an X-ray generator (106) of the tomography apparatus which has a radiation focal point.

2. The tomography apparatus of claim 1, wherein the controller (118) is configured to estimate a PSF corresponding to a channel in which the object is located among a plurality of channels formed between an X-ray generator (106) and an X-ray detector (108).

3. The tomography apparatus of claim 1, wherein the controller (118) is configured to estimate the PSF based on projection data of a sample object and geometric information of the sample object.

4. The tomography apparatus of claim 3, wherein the geometric information comprises outline information of the sample object.

5. The tomography apparatus of claim 1, wherein the image processor (126) is adapted to perform de-blurring on projection data scanned at a rotation angle of a gantry (102).

6. The tomography apparatus of claim 5, wherein the image processor (126) is further adapted to obtain a plurality of projection data corresponding to a plurality of rotation angles, and to perform de-blurring on the projection data corresponding to each rotation angle.

7. The tomography apparatus of claim 1, wherein the controller (118) is further configured to determine a distance between the X-ray generator (106) and the object (10) based on a cross-sectional image of a sample object scanned at a position.

8. The tomography apparatus of claim 2, wherein the controller (118) is further configured to determine a channel in which the object is located among a plurality of channels formed between the X-ray generator (106) and the X-ray detector (108) based on a cross-sectional image of a sample object scanned at a position.

9. The tomography apparatus of claim 1,
further comprising: a storage (124) for storing the PSF,
wherein the storage (124) is adapted to store a plurality of PSFs mapped to different positions of the object.

10. A method for controlling a tomography apparatus (100) comprising:
estimating a Point Spread Function (PSF) corresponding to each location of an object (10); and

de-blurring projection data of the object (10) based on the PSF corresponding to the location of the object, **characterised in that** the step of estimating a PSF corresponding to each location of an object comprises estimating a PSF corresponding to a distance between the object (10) and an X-ray generator (106) of the tomography apparatus which has a radiation focal point.

11. The method of claim 10,
wherein a distance between the X-ray generator (106) and the object (10) is determined based on a cross-sectional image of a sample object scanned at a position.

12. The method of claim 10,
wherein estimating a PSF corresponding to each location of an object (10) comprises estimating a PSF corresponding to a channel in which the object is located among a plurality of channels formed between an X-ray generator (106) and an X-ray detector (108).

13. The tomography apparatus of claim 1,
further comprising: an adaptive filter (515) for correcting an PSF estimated by the controller (118),
wherein the adaptive filter (515) is configured to correct the PSF depending on a location of a region of interest.

14. The tomography apparatus of claim 13,
wherein the PSF estimated by the controller (118) is a first PSF, and
wherein the adaptive filter (515) is configured to generate a second PSF based on a distance from a center of a Field of View (FOV) to the region of interest and a distance from a focal point of X-ray radiation.

**Patentansprüche**

1. Tomographievorrichtung (100), umfassend:

eine Steuerung (118) zum Schätzen einer Punktstreufunktion (PSF), die einem Standort eines Objekts (10) entspricht; und
einen Bildprozessor (126) zum Beseitigen von Unschärfe in den Projektionsdaten des Objekts (10) auf der Basis der PSF, die dem Standort des Objekts (10) entspricht,
**dadurch gekennzeichnet, dass**
die Steuerung (118) dazu ausgestaltet ist, die PSF, die einem Abstand zwischen dem Objekt und einem Röntgenstrahlerzeuger (106) der Tomographievorrichtung, der einen Strahlungsbrennpunkt aufweist, entspricht, zu schätzen.

2. Tomographievorrichtung nach Anspruch 1,
wobei die Steuerung (118) dazu ausgestaltet ist, eine PSF, die einem Kanal, in welchem sich das Objekt befindet, aus einer Mehrzahl von Kanälen, die zwischen einem Röntgenstrahlerzeuger (106) und einem Röntgenstrahldetektor (108) ausgebildet sind, entspricht, zu schätzen.

3. Tomographievorrichtung nach Anspruch 1,
wobei die Steuerung (118) dazu ausgestaltet ist, die PSF auf der Basis eines Musterobjekts und geometrischer Informationen des Musterobjekts zu schätzen.

4. Tomographievorrichtung nach Anspruch 3,
wobei die geometrischen Informationen Umrissinformationen des Musterobjekts umfassen.

5. Tomographievorrichtung nach Anspruch 1,
wobei der Bildprozessor (126) dazu angepasst ist, eine Beseitigung von Unschärfe in den Projektionsdaten, die in einem Drehungswinkel einer Gantry (102) gescannt wurden, auszuführen.

**6.** Tomographievorrichtung nach Anspruch 5,
wobei der Bildprozessor (126) ferner dazu angepasst ist, eine Mehrzahl von Projektionsdaten, die einer Mehrzahl von Drehungswinkeln entsprechen, zu erhalten, und eine Beseitigung von Unschärfe in den Projektionsdaten, die jedem Drehungswinkel entsprechen, auszuführen.

**7.** Tomographievorrichtung nach Anspruch 1,
wobei die Steuerung (118) ferner dazu ausgestaltet ist, einen Abstand zwischen dem Röntgenstrahlerzeuger (106) und dem Objekt (10) auf der Basis eines Querschnittsbilds eines Musterobjekts, das an einer Position gescannt wurde, zu bestimmen.

**8.** Tomographievorrichtung nach Anspruch 2
wobei die Steuerung (118) ferner dazu ausgestaltet ist, einen Kanal, in welchem sich das Objekt befindet, aus einer Mehrzahl von Kanälen, die zwischen dem Röntgenstrahlerzeuger (106) und dem Röntgenstrahldetektor (108) ausgebildet sind, auf der Basis eines Querschnittsbilds eines Musterobjekts, das an einer Position gescannt wurde, zu bestimmen.

**9.** Tomographievorrichtung nach Anspruch 1,
ferner umfassend: einen Speicher (124) zum Speichern der PSF,
wobei der Speicher (124) dazu ausgelegt ist, eine Mehrzahl von PSFs, die verschiedenen Positionen des Objekts zugeordnet sind, zu speichern.

**10.** Verfahren zum Steuern einer Tomographievorrichtung (100), umfassend:

Schätzen einer Punktstreufunktion (PSF), die jedem Standort eines Objekts (10) entspricht; und
Beseitigen von Unschärfe in den Projektionsdaten des Objekts (10) auf der Basis der PSF, die dem Standort des Objekts entspricht,
**dadurch gekennzeichnet, dass**
der Schritt des Schätzens einer PSF, die jedem Standort eines Objekts entspricht, das Schätzen einer PSF, die einem Abstand zwischen dem Objekt (10) und einem Röntgenstrahlerzeuger (106) der Tomographievorrichtung, der einen Strahlungsbrennpunkt aufweist, entspricht, umfasst.

**11.** Verfahren nach Anspruch 10,
wobei ein Abstand zwischen dem Röntgenstrahlerzeuger (106) und dem Objekt (10) auf der Basis eines Querschnittsbilds eines Musterobjekts, das an einer Position gescannt wurde, bestimmt wird.

**12.** Verfahren nach Anspruch 10,
wobei das Schätzen einer PSF, die jedem Standort eines Objekts (10) entspricht, das Schätzen einer PSF, die einem Kanal, in welchem sich das Objekt befindet, aus einer Mehrzahl von Kanälen, die zwischen einem Röntgenstrahlerzeuger (106) und einem Röntgenstrahldetektor (108) ausgebildet sind, entspricht, umfasst.

**13.** Tomographievorrichtung nach Anspruch 1,
ferner umfassend: einen anpassbaren Filter (515) zum Korrigieren einer durch die Steuerung (118) geschätzten PSF,
wobei der anpassbare Filter (515) dazu ausgestaltet ist, die PSF in Abhängigkeit von einem Standort eines interessierenden Abschnitts zu korrigieren.

**14.** Tomographievorrichtung nach Anspruch 13,
wobei die durch die Steuerung (118) geschätzte PSF eine erste PSF ist, und
wobei der anpassbare Filter (515) dazu ausgestaltet ist, eine zweite PSF auf der Basis eines Abstands von einem Zentrum eines Blickfelds (FOV) zum interessierenden Abschnitt und eines Abstands von einem Brennpunkt von Röntgenstrahlung zu erzeugen.

**Revendications**

**1.** Appareil de tomographie (100) comprenant :

un dispositif de commande (118) destiné à estimer une fonction d'étalement du point (PSF) correspondant à un emplacement d'un objet (10) ; et

un processeur d'images (126) destiné à corriger le flou de données de projection de l'objet (10) sur la base de la PSF correspondant à l'emplacement de l'objet (10),

**caractérisé en ce que**

le dispositif de commande (118) est configuré pour estimer la PSF correspondant à une distance entre l'objet et un générateur de rayons X (106) de l'appareil de tomographie, qui présente un foyer de rayonnement.

2. Appareil de tomographie selon la revendication 1,
dans lequel le dispositif de commande (118) est configuré pour estimer une PSF correspondant à un canal dans lequel l'objet est situé parmi une pluralité de canaux formés entre un générateur de rayons X (106) et un détecteur de rayons X (108).

3. Appareil de tomographie selon la revendication 1,
dans lequel le dispositif de commande (118) est configuré pour estimer la PSF sur la base de données de projection d'un objet échantillon et d'informations géométriques de l'objet échantillon.

4. Appareil de tomographie selon la revendication 3,
dans lequel les informations géométriques comprennent des informations de contour de l'objet échantillon.

5. Appareil de tomographie selon la revendication 1,
dans lequel le processeur d'images (126) est conçu pour réaliser une correction du flou sur des données de projection balayées selon un angle de rotation d'un portique (102).

6. Appareil de tomographie selon la revendication 5,
dans lequel le processeur d'images (126) est en outre conçu pour obtenir une pluralité de données de projection correspondant à une pluralité d'angles de rotation, et pour effectuer une correction du flou sur les données de projection correspondant à chaque angle de rotation.

7. Appareil de tomographie selon la revendication 1,
dans lequel le dispositif de commande (118) est en outre configuré pour déterminer une distance entre le générateur de rayons X (106) et l'objet (10) sur la base d'une image de section transversale d'un objet échantillon balayé dans une position.

8. Appareil de tomographie selon la revendication 2,
dans lequel le dispositif de commande (118) est en outre configuré pour déterminer un canal dans lequel l'objet est situé parmi une pluralité de canaux formés entre le générateur de rayons X (106) et le détecteur de rayons X (108) sur la base d'une image de section transversale d'un objet échantillon balayé dans une position.

9. Appareil de tomographie selon la revendication 1,
comprenant en outre : une mémoire (124) pour stocker la PSF,
dans lequel la mémoire (124) est conçue pour stocker une pluralité de PSF attribuées à différentes positions de l'objet.

10. Procédé de commande d'un appareil de tomographie (100), comprenant :

l'estimation d'une fonction d'étalement du point (PSF) correspondant à chaque emplacement d'un objet (10) ; et
la correction du flou de données de projection de l'objet (10) sur la base de la PSF correspondant à l'emplacement de l'objet,
**caractérisé en ce que**
l'étape d'estimation d'une PSF correspondant à chaque emplacement d'un objet comprend l'estimation d'une PSF correspondant à une distance entre l'objet (10) et un générateur de rayons X (106) de l'appareil de tomographie, qui présente un foyer de rayonnement.

11. Procédé selon la revendication 10,
dans lequel une distance entre le générateur de rayons X (106) et l'objet (10) est déterminée sur la base d'une image de section transversale d'un objet échantillon balayé dans une position.

12. Procédé selon la revendication 10,

dans lequel l'estimation d'une PSF correspondant à chaque emplacement d'un objet (10) comprend l'estimation d'une PSF correspondant à un canal dans lequel l'objet est situé parmi une pluralité de canaux formés entre un générateur de rayons X (106) et un détecteur de rayons X (108).

13. Appareil de tomographie selon la revendication 1,
comprenant en outre : un filtre adaptatif (515) destiné à la correction d'une PSF estimée par le dispositif de commande (118),
dans lequel le filtre adaptatif (515) est configuré pour corriger la PSF en fonction d'un emplacement d'une région d'intérêt.

14. Appareil de tomographie selon la revendication 13,
dans lequel la PSF estimée par le dispositif de commande (118) est une première PSF, et
dans lequel le filtre adaptatif (515) est configuré pour générer une seconde PSF sur la base d'une distance d'un centre d'un champ de vue (FOV) à la région d'intérêt et d'une distance depuis un foyer de rayonnement de rayons X.

[Fig. 1]

[Fig. 2]

[Fig. 3]

[Fig. 4a]

Deconvolution with inaccurate PSF

[Fig. 4b]

Deconvolution without noise modeling

[Fig. 5a]

500

510                          520

CONTROLLER  ⟷  IMAGE PROCESSOR

530

STORAGE

[Fig. 5b]

106

501                          511

$B_1$                                          $y_1$   512

$B_2$                                          $y_2$   513

$B_3$                                          $y_3$

114

[Fig. 5c]

[Fig. 6]

[Fig. 7]

[Fig. 8a]

[Fig. 8b]

[Fig. 8c]

730

I

PSF

740

$\circledast$

750

$I * PSF$

[Fig. 9]

800    803  802    801

$\Rightarrow$

HU

810

811

812

800
700
600
500
400
300
200
100
0
-100

0   5   10   15   20   25   30   35

X

[Fig. 10a]

900

y or r

901

902

$y_6$ or $r_6$

$y_5$ or $r_5$

903

$y_4$ or $r_4$

$y_3$ or $r_3$

$y_2$ or $r_2$

$y_1$ or $r_1$

$x_1$   $x_2$   $x_3$   $x_4$   $x_5$   X

[Fig. 10b]

910

911

912

$y_5$ or $r_5$

$y_4$ or $r_4$

$y_3$ or $r_3$

$y_2$ or $r_2$

$y_1$ or $r_1$

[Fig. 10c]

920

ROI 1

y or r

$y_6$ or $r_6$

$y_5$ or $r_5$

921

$y_4$ or $r_4$

922

$y_3$ or $r_3$

$y_2$ or $r_2$

$y_1$ or $r_1$

ROI 2  $x_1$  $x_2$  $x_3$  X

[Fig. 11]

[Fig. 12]

[Fig. 13]

$(x_1 , y_1 )$

—————————————————— 0°

$(x_2 , y_2 )$

—————————————————— 30°

.
.
.
.
.
.

—————————————————— 360°

[Fig. 14]

[Fig. 15]

```
            ┌─────────┐
            │  START  │
            └─────────┘
                 │
                 ▼
   ┌──────────────────────────────────┐
   │      ESTIMATE MULTIPLE PSF        │── S1210
   │  CORRESPONDING TO MULTIPLE POSITION│
   └──────────────────────────────────┘
                 │
                 ▼
   ┌──────────────────────────────────┐
   │   PERFORM DE-BLURRING BASED ON PSF │── S1220
   │   CORRESPONDING TO POSITION OF     │
   │ OBJECT AND PROJECTION DATA OF OBJECT│
   └──────────────────────────────────┘
                 │
                 ▼
   ┌──────────────────────────────────┐
   │        RECONSTRUCT IMAGE          │── S1230
   └──────────────────────────────────┘
                 │
                 ▼
            ┌─────────┐
            │   END   │
            └─────────┘
```

[Fig. 16]

```
            ┌─────────┐
            │  START  │
            └─────────┘
                 │
                 ▼
   ┌──────────────────────────────────┐
   │    OBTAIN CROSS-SECTIONAL IMAGE   │── S1310
   │        OF SAMPLE OBJECT           │
   └──────────────────────────────────┘
                 │
                 ▼
   ┌──────────────────────────────────┐
   │    DETERMINE BAND OR BEAM LINE IN │── S1320
   │    WHICH SAMPLE OBJECT IS LOCATED │
   └──────────────────────────────────┘
                 │
                 ▼
   ┌──────────────────────────────────┐
   │     ESTIMATE PSF BASED ON OUTLINE │── S1330
   │  INFORMATION AND PROJECTION DATA  │
   │          OF SAMPLE OBJECT         │
   └──────────────────────────────────┘
                 │
                 ▼
   ┌──────────────────────────────────┐
   │      STORE BAND OR BEAM LINE      │── S1340
   │        INFORMATION AND PSF        │
   └──────────────────────────────────┘
                 │
                 ▼
            ┌─────────┐
            │   END   │
            └─────────┘
```

[Fig. 17]

```
                    ┌─────────────┐
                    │    START    │
                    └──────┬──────┘
                           │                                          ┌──────────────────────┐
                           ▼                                          │                      │
S1410   ┌──────────────────────────────────┐                         │                      │
        │   OBTAIN CROSS-SECTIONAL          │                         │                      │
        │   IMAGE OF OBJECT                 │                         │                      │
        └──────────────────┬───────────────┘                         │                      │
                           │                                          │                      │
                           ▼                                          │                      │
S1420   ┌──────────────────────────────────┐                         │                      │
        │   DETERMINE BAND OR BEAM LINE     │                         │                      │
        │   IN WHICH OBJECT IS LOCATED      │                         │                      │
        └──────────────────┬───────────────┘                         │                      │
                           │                                          │                      │
                           ▼                                          │                      │
        ┌──────────────────────────────────┐                         │                      │
        │   PERFORM DEBLURRING ON           │                         │                      │
        │   PROJECTION DATA OF OBJECT       │                         │                      │
S1430   │   BASED ON PSF CORRESPONDING      │                         │                      │
        │   TO BAND OR BEAM LINE IN         │                         │                      │
        │   WHICH OBJECT IS LOCATED         │                         │                      │
        └──────────────────┬───────────────┘                         │                      │
                           │                                          │                      │
                           ▼                                          │                      │
                    ╱────────────╲                          S1460     │                      │
S1440          ╱  ROTATION ANGLE  ╲      NO                           │                      │
              ╲  = 360 DEGREES?   ╱────────────┐    ┌─────────────────────────────────┐      │
               ╲────────────────╱              └───▶│   PERFORM SCANNING AT           │──────┘
                       │ YES                         │   NEXT ROTATION ANGLE           │
                       ▼                             └─────────────────────────────────┘
S1450   ┌──────────────────────────────────┐
        │   RECONSTRUCT IMAGE BASED ON      │
        │   MULTIPLE PROJECTION DATA        │
        └──────────────────┬───────────────┘
                           │
                           ▼
                    ┌─────────────┐
                    │     END     │
                    └─────────────┘
```

**EP 3 349 655 B1**

**Patent documents cited in the description**

- EP 3326533 A1
- US 20130182928 A1